(19) Europäisches Patentamt / European Patent Office / Office européen des brevets

(11) **EP 3 669 941 B1**

(12) **EUROPEAN PATENT SPECIFICATION**

(45) Date of publication and mention of the grant of the patent:
**12.07.2023 Bulletin 2023/28**

(21) Application number: **18214842.9**

(22) Date of filing: **20.12.2018**

(51) International Patent Classification (IPC):
**A61N 5/10** (2006.01)          **G21K 5/04** (2006.01)

(52) Cooperative Patent Classification (CPC):
**A61N 5/103; A61N 5/1045;** A61N 2005/1087;
A61N 2005/1095

(54) **SYSTEM AND METHOD FOR PLANNING OF PASSIVE ION RADIOTHERAPY TREATMENT**

SYSTEM UND VERFAHREN ZUR PLANUNG EINER PASSIVEN IONENSTRAHLENTHERAPIEBEHANDLUNG

SYSTÈME ET PROCÉDÉ DE PLANIFICATION DE TRAITEMENT PAR RADIOTHÉRAPIE IONIQUE PASSIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.06.2020 Bulletin 2020/26**

(73) Proprietor: **RaySearch Laboratories AB**
**104 30 Stockholm (SE)**

(72) Inventors:
• **Fredriksson, Albin**
  **11869 Stockholm (SE)**
• **Engwall, Erik**
  **129 44 Hägersten (SE)**

(56) References cited:
**EP-A1- 3 108 932      US-A1- 2017 281 974**
**US-B2- 7 834 336**

**Description**

Technical Field

The present invention relates to a system and a method for planning of passive ion radiotherapy treatment, in which a tumor is targeted with a beam of ions (e.g. protons or heavier ions like carbon).

Background

Radiotherapy is commonly used for treating diseases such as cancer. Various types of radiation sources may be used. The predominant type of radiation used today is photon radiation. Although more expensive than photon radiotherapy, ion-based therapy methods, such as proton and carbon ion therapy, are becoming more common because of their advantages. In particular, the deposition of dose can be controlled more precisely because an ion beam has a finite range determined by its initial energy. Also, an ion will emit most of its energy close to the depth where it stops, in the area known as the Bragg peak.

Passive ion therapy involves applying a broad field of radiation and using physical elements, referred to as passive devices, to shape the beam to match the target as precisely as possible. The beam energy will control the maximum range of the ions in the patient. The lateral shape of the beam is controlled by an element defining an aperture in the beam line, such as a block or a collimator of a non-permeable material, for example tungsten or brass. The width of the high dose region, i.e. the Spread Out Bragg Peak (SOBP), is controlled by for example a range modulator or ridge filter. Typically, a range compensator is also used, which is placed in the beam trajectory to affect the maximum depth of the beam differently in different lateral positions in the patient's anatomy. The compensator shortens the local range of the ion beam and is non-uniformly shaped to conform to the distal edge of the tumour. Traditionally, the settings for a passive ion therapy plan, that is, the shape of the aperture, the range and width of the SOBP and the shape of the compensator, are created by forward planning.

European patent application EP 15173971 relates to the planning of passive ion radiotherapy treatment by optimization and discloses the main components of a delivery system. The basic principle is that the settings for passive ion therapy plans are determined through inverse planning using optimization objectives. The aim of this prior application is to find the optimal range and width of the SOBP for each beam angle, as well as to design the block to achieve the correct dose distribution. Document US 2017/281974 discloses an radiotherapy device using an ion beam, the beam being modulated range modulation wheels and MLCs. Optimisation is performed wherein MLC settings can be set individually for each gantry angle; settings of the wheels can be varied during irradiation from a specific gantry angle.

Optimization is used as a standard in active scanning ion techniques, such as pencil-beam scanning. In this type of delivery, a large number of small (pencil) beams, also referred to as spots, are used to cover the tumor in all three dimensions. The spots are grouped into different energy layers to reach different depths of the tumor. Within the energy layers the spots are distributed laterally by scanning magnets in the beam line. The desired dose distribution is achieved through optimization whereby the intensities of the spots are altered.

There is currently a trend to focus more on active techniques rather than passive techniques. The main reason is that it is easier to obtain a conformal dose distribution with active scanning techniques at the same time as patient-specific hardware, such as compensators and block apertures, are not needed.

Summary

It is an object of the present invention to improve the planning of passive, ion-based treatment plans.

The invention relates to a method of optimizing a passive radiotherapy treatment plan for ion therapy designed to expose a patient to ion radiation delivered as at least a first beam from at least a first beam angle, where each beam is shaped by means of passive devices including: a range modulating device for creating a spread out Bragg peak, SOBP and an aperture element for shaping the beam laterally, using an optimization problem set up to allow variation in settings of the range modulating device, and settings of the aperture element, both during the delivery of the first beam, so that said plan will include modulation of the fluence of the beam during the delivery of the beam. The settings of the range modulating device typically include one or more of the following: current, energy and delivery times.

In a preferred embodiment, the optimization problem is set up in such a way that the resulting radiotherapy treatment plan will be designed to deliver ion radiation in at least a first beam and a second beam, from at least a first and a second beam angle, respectively, the method including the step of modulating the fluence of each beam during the delivery of the beam.

The optimization problem is set up to allow variation of the settings of the aperture element during the delivery of the at least first beam, so that said plan will include lateral modulation of the beam during the delivery of the beam. This will enable lateral modulation of the beam by variation of the aperture. The aperture element may be an MLC, jaws or an iris diaphragm, or any other variable aperture shaping element suitable for ion radiotherapy. If an MLC is used, it may be controlled to function as a static MLC, a dynamic MLC or in some other way.

If an MLC is used, and is controlled as a static MLC, the optimization problem may be set up to optimize at least the first beam for delivery in at least a first and a second segment and to return a plan where the settings for the aperture element are different for the first and second segment.

If an MLC is used, and is controlled as a dynamic MLC, the optimization problem may be set up to optimize the at least first beam wherein control points are used to define at least a first and a second portion of the at least first beam and the settings for the aperture element are different for the first and the second portion.

In addition to controlling the aperture element, the optimization problem is set up to allow variation of the range modulating device during the delivery of the at least first beam, so that said plan will include modulation in depth of the beam during the delivery of the beam. This enables variation of the depth profile of the dose during delivery of the beam. The range modulating device may be a ridge filter or a range modulator wheel, or any other suitable device for modulating the beam range. The SOBP may be shaped to have a flat shape as is common in the art, but may also be given any other suitable shape.

The optimization problem may be set up to optimize at least the first beam for delivery in at least a first and a second segment and to return a plan where the settings for the range modulating device are different for the first and second segment. Alternatively, the optimization problem is set up to optimize the at least first beam wherein control points are used to define at least a first and a second portion of the at least first beam and the settings for the range modulating device are different for the first and the second portion. This will depend on how the MLC is controlled, as discussed above.

The invention also relates to a computer program product comprising computer readable code means, which, when run in a computer, will cause the computer to perform the method according to any one of the preceding claims.

The invention also relates to a computer system for performing dose calculations for radiotherapy, the system comprising processing means, said computer system having a program memory having stored therein a computer program product as outlined above, in such a way that the computer program product, when executed, will control the processing means.

The invention also relates to a radiotherapy delivery system for delivering a passive ion plan wherein ions are delivered to a patient in at least a first beam from at least a first beam angle, the system comprising a range modulating device for creating a spread-out Bragg peak, SOBP, and an aperture element for shaping the beam laterally, the system further comprising control means for varying the settings of the aperture element and the settings of the range modulating device, both during delivery of the at least first beam, so as to modulate the fluence of the beam during delivery of the beam.

The aperture element comprises an element that is capable of varying the lateral shape of the beam, in which case the control means should be arranged to control the aperture element so as to modulate the at least one beam laterally by using multiple settings of the aperture element for at least one beam angle.

The control means is further arranged to control the range modulating device to vary the SOBP width in such a way that at least two different SOBT widths are applied to the same beam, to enable modulation of the depth profile of the beam.

As is common in the art, the system may further comprise a range compensator and any other suitable components.

The optimization preferably includes optimization functions based on dose levels, but could additionally include optimization functions related to any other relevant quantities, such as LET or the actual track ends where the ion stops. The method can be combined with any suitable method of robust optimization. It may also be combined with multi-criteria optimization.

Brief Description of the Drawings

The invention will be described in more detail in the following, by way of example and with reference to the appended drawings, in which

Figure 1 shows an example of a system for passive ion therapy.
Figure 2 illustrates an SOBP.
Figure 3 is a flow chart of the planning method.
Figure 4 illustrates a computer system that may be used in dose planning according to the invention.

Detailed Description

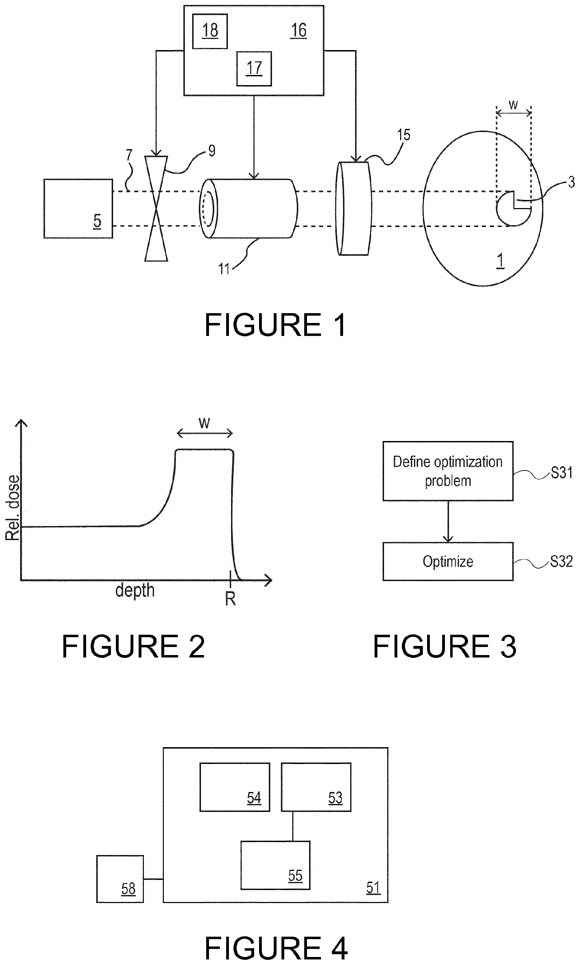

Fig. 1 shows schematically an example of a delivery system for passive ion therapy in which the invention may be implemented. A patient 1 that is to be subjected to ion therapy is shown schematically to the right in Fig. 1. A region of interest ROI, or target 3, within the patient 1 represents the organ or other tissue that is to receive the radiotherapy. The maximum width of the target 3 is marked as w. As is common in the art, there may also be defined critical areas within the patient, which are areas in which it is particularly important to avoid radiation, although this is not shown in Fig. 1.

A radiation source 5 provides an ion beam 7 having sufficient energy to achieve the desired maximum range, typically reaching to the distal target 3 edge. Typically, one or two scattering devices (not shown) is arranged to create a broad field of radiation. Alternatively, a uniform scanning technique or wobbling may be used to create a broad field. In passive ion therapy, the dose is subsequently shaped to the target, that is, the region of interest, using passive devices. First, in the path of the radiation, a range modulating device 9 is arranged, in order to create the SOBP as will be discussed in more detail in connection with Fig. 4. In short, the range modulating device 9 determines the width w (along the beam direction) of the SOBP, which should be wide enough to cover the area of the target 3. After the range modulating device 9 a beam shaping device 11, shown in Fig. 1 as a block 11 is arranged to shape the beam laterally. The block 11, typically made from brass or some other material that will not be penetrated by the ions, has an aperture 13 for letting the beam through. Instead of the block, another type of device suitable for laterally shaping the beam may be used. Suitable devices, as will be discussed in more detail below, include multi-leaf collimators (MLC), jaws, iris diaphragms. The MLC may be controlled to function as a segmented MLC (SMLC, sometimes called a static MLC), or as a dynamic MLC (DMLC), or in another suitable way, depending on the characteristics of the delivery system.

The beam energy is chosen so that the maximum ion beam range agrees with the most distal point on the target. Of course, more complex patient geometries and target shapes often occur, and the range will be too large for at least some of the target. To compensate for the variation in water equivalent distance to the distal end of the tumour, a range compensator 15 is introduced to control the local range over the cross-section of the ion beam. Although this is not shown in Fig. 1, the thickness of the range compensator varies across the beam trajectory in a way well known in the art to adapt the beam energy to the distal end of the target in each point.

The range modulating device 9 is arranged to form an SOBP. Such range modulating devices are known in the art, including ripple filters and range modulator wheels, which are rotating disks with variable thickness. Co-pending application (EP18183065.4) discloses a ripple filter that can be varied dynamically without having to change filters.

According to the invention a patient is exposed to radiation provided from at least one angle, preferably a number of different angles, known as beam angles. This may be achieved in any suitable manner, by rotating the gantry, or by moving the chair or couch on which the patient is positioned.

The invention is based on the insight that the ion-based radiotherapy treatment can be further improved if the treatment plan includes variation of the delivery parameters enabling modulation within each beam. To achieve this, the delivery system further comprises control means 16 arranged to control at least one of the range modulating device 9 and the lateral beam shaping device 11. The control involves changing the settings of either the range modulating device 9 or the lateral beam shaping device 1 1or both of them during the delivery of one particular beam angle, to apply at least two different lateral modulations by varying the settings of the lateral beam shaping device 11 and/or two different modulations in depth by varying the settings of the range modulating device 9.

Fig. 4 illustrates schematically the SOBP of a proton beam within a patient in terms of the relative dose as a function of depth. As can be seen, the dose increases from a plateau region to a maximum, which is constant over a distance w. After the SOBP the dose will fall to zero within a short distance. The maximum range of the beam is denoted as R. The maximum emitted energy should occur when the proton beam travels through the target, after which the emitted energy should fall to zero as soon as possible. The depth dose shape for other ions is similar to the proton case, except that there will be a low dose tail after the SOBP resulting from nuclear fragments.

To improve the radiotherapy plan, the range of the beam and the design of the passive devices, that is, the range modulating device 9, the lateral beam shaping device 11 and the range compensator 15, may be varied. Traditionally this is done by forward planning methods, by computing the design of the passive devices through ray tracing of the patient geometry and the parameters are kept constant within each beam. To enable the optimization of ion-based treatment plans allowing the modulation of each beam, the optimization problem must be designed to allow different settings of range modulating device and aperture element during the delivery of each beam. How to implement this depends on the elements used, that is, the setup of the delivery system. This will be discussed for the different elements in the following.

The lateral beam shaping device 11 may be a MLC. When there are multiple MLC positions for at least one beam in the treatment, this results in lateral modulation of the beam. The MLC leaf positions can be included as variables in the optimization, which allows the optimization algorithm to determine the modulation that results in the most beneficial dose distribution for the patient, as measured by some at least partly dose-based function that measures the quality of the dose.

In static collimation, using an SMLC, each beam is delivered from its beam angle as a number of segments. Each segment is defined by an aperture shape, given by the leaf position settings of the MLC, and a segment MU, which is proportional to the time the beam delivers radiation through the aperture. Preferably, lateral modulation of the beam is achieved in the following way: when a segment has been delivered, the beam is turned off, and the MLC leaves move to form a new aperture, after which the beam is turned on to deliver the next segment. Therefore, according to this embodiment, the MLC leaves do not move while the beam is on. The total fluence delivered from a beam is the sum of the fluences of its segments.

In dynamic collimation, using a DMLC, the MLC leaves move while the beam is on. Typically, but not necessarily, the leaves sweep from one side of the field to the other. This can yield a highly modulated field. If the leaves sweep from left to right and the dose rate is denoted by d, the point in time at which the right leaf exposes a given point is R, and the point in time at which the left leaf covers the point is L, then the fluence delivered from the point is d(L-R). In general, for a variable dose rate, the fluence from the point is the integral of the dose rate from R to L. Since R and L can be changed without much restriction (the restrictions for the leaves being the maximum leaf speed and the maximally allowed treatment time), almost any fluence map can be delivered.

When using both DMLC and SMLC, control points are used to communicate the specifics of the treatment plan to the machine. For SMLC treatments, a segment is defined as two control points with identical leaf positions but different cumulative MU.

For all types of lateral beam shaping devices with variable apertures, it may be suitable to include in the optimization problem constraints on how much the beam shaping device is allowed to change between two consecutive control points, to limit the complexity of the plan, in terms of the degree of intensity modulation. A higher degree of modulation results in a plan that is more sensitive to the uncertainties in the setup. A lower degree of modulation also results in shorter treatment duration, which is advantageous. It is also possible to include constraints on how much MU is allowed to vary between control points. In particular, it is undesirable to have many segments with low MU, as this may make the plan sensitive to interplay effects. It may also be feasible to include constraints on the minimum segment area, in absolute or relative terms. A high limit on the minimum segment area will result in less modulation, since a larger portion of the target will be covered by each segment.

According to embodiments of the invention, the shape of the SOBP is optimized to enable modulation in depth, by varying the settings of the range modulating device 9 during delivery of one beam. The SOBP is built up of multiple individual Bragg peaks. Each of these can be controlled by a weight. Modulation in depth is achieved when the weights of the individual Bragg peaks are included as variables in the optimization in such a way that the weights are allowed to vary during the delivery of one beam. As is the case for the lateral modulation, modulation in depth may be achieved by using different sets of variables for different segments or by allowing the sets of variables to be changed at control points.

Instead of optimizing the weight of each Bragg peak individually, the range and width of each SOBP may be optimized in such a way that the relationship between the weights of the Bragg peaks within the range and the width is predetermined but the range and the width may change. The relationship between the weights is preferably set in such a way that the SOBP will be flat in the interval between the range and the width. To achieve this, a predetermined set of weights for the SOBP may be used, while all weights for Bragg peaks outside of the range and width may be set to 0. In this case, a range and width setting per segment or control point will result in a modulation in depth.

Modulation in depth can also be achieved if optimization of the full shape of the SOBP is performed, in which the weights of the individual Bragg peaks are allowed to vary freely. In this case the shape of the SOBP may be determined per plan, per beam or, as above, per segment or control point.

It may also be feasible to include one or more constraints in the optimization problem limiting the modulation of the SOBP. For example, constraints may be added to limit how much adjacent weights of the SOBP are allowed to differ from each other, or from the predetermined weights that result in a flat SOBP.

As is known in the art, optimization involves solving an optimization problem, which has the general format shown in Eq. (1).

$$\min f(x) \qquad (1)$$

$$\text{st } x \in X$$

where f(x) is a quality measure of the variables x and X is the set of allowed variable values.

The skilled person is aware of a number of ways of measuring the quality, for example based on the physical dose or on biological models. As one example, the f(x) can be

$$f(x) = g(d(x)) \qquad (2)$$

where d(x) is the dose as a function of the variables x and g measures the quality of the dose, for example as

$$\sum_i w_i \left( d_i - d_i^{ref} \right)^2, \qquad (3)$$

where $d_i^{ref}$ is a desired dose level for voxel i and w; is the importance weight of voxel i.

As the skilled person is aware, it would also be possible to consider functions that are dependent on x but not the dose, or that are dependent on both x and the dose, for example as

$$f(x) = g(d(x)) + h(x) \qquad (4)$$

where h is a different quality measure for x, for example, the smoothness of the shape of the MLC or the time required for changing the range and width between neighboring segments.

An example for the set X, given that x = (r, w) are the range and width for the SOBP

$$X = \{ (r,w) : r_{min} \leq r \leq r_{max}, w_{min} \leq w \leq w_{max} \} \qquad (5)$$

Alternatively, if x = (l, r) are the positions of the left and right leaves, respectively, of an MLC, X could be:

$$X = \{(l,r) : l_{min} \leq l \leq l_{max}, r_{min} \leq r \leq r_{max}, l + m \leq r \} \qquad (6)$$

where m is the minimum gap between the left and right leaves.

As the skilled person is aware, there are a number of possible limitations and the ones mentioned above serve only as examples. Other possible parameters include interdigitation and max tip difference. Typically, x will comprise more variables than r, w or l, r, including such variables as segment weights or control point MU.

When the optimization problem has been set up, it can be solved in different ways, which are known in the art. Such methods include gradient based methods, using information about the gradient of f with respect to x to determine how x should change, and stochastic method such as simulated annealing or genetic algorithms.

The optimization discussed above is machine parameter optimization, in which the parameters that will control the delivery system are optimized directly. Typically, a pre-calculation step is performed to get to a situation where optimization can be performed on segments, control points or SOBPs. The pre-calculation step often involves optimization based on a possibly unattainable fluence map, in which each bixel weight is allowed to vary individually, that is subsequently converted to segments or control points with machine parameters. A bixel is defined as a small intensity element used to subdivide an intensity-modulated beam for intensity distribution optimization or dose calculations during treatment planning.

The conversion of a fluence map into deliverable segments or control points can be performed in various ways that are known in the art. For example, for delivery using an SMLC technique, the fluence map can be discretized into a number of levels, for example using equidistant levels or using clustering to determine levels with varying distances. Each level of the discretized fluence map can then be converted into a number of segments satisfying the machine constraints.

Alternatively, one could use column generation methods (sometimes called direct aperture optimization), in which segments are generated iteratively. For each beam, the gradient of the fluence map can be used to calculate which segment shape satisfying the machine constraint would locally improve the solution the most. That segment is then added to the current set of segments, and a segment weight optimization is performed.

For delivery using a DMLC technique, conversion from a fluence map into machine parameters can typically be performed analytically or using linear programming. The weight of each bixel then determines the difference between the times at which the right and the left leaves, respectively, begin traversing the bixel.

A number of additional features can be combined with the setup according to the invention. For moving targets, the adjustable aperture of the lateral beam shaping device can also be used to follow the target. Also, robust objective functions can be added in the optimization of the treatment plan. Robustness could be based on uncertainties in setup, range (CT to density interpretation), patient geometry (organ motion) or in the delivery time structures (interplay).

The multiple degrees of freedom introduced by the variables corresponding to the MLC and the SOBP allows for a wide variety of treatments. To allow the user to explore the options, multicriteria optimization (MCO) in the form of Pareto surface navigation may be used. Pareto optimization in this context is discussed in Thieke et al. A new concept for interactive radiotherapy planning with multicriteria optimization: First clinical evaluation, Radiotherapy and Oncology 85(2), 292-298, 2007 and Craft et al. An approach for practical multiobjective IMRT treatment planning, International Journal of Radiation Oncology*Biology*Physics, 69(5), 1600-1607, 2007). Such multi-criteria optimization can be done for all the approaches above. The method works by taking weighted sums of the dose distributions of precalculated plans. When a desirable combination has been found, a deliverable plan must be calculated. It is typically calculated in a mimicking step, in which an optimization is performed to replicate the navigated dose. The resulting plan not always replicate the navigated dose exactly. However, some treatment techniques are well suited for combining plans, and results in directly deliverable plans without the need for a degrading conversion. This has been described for photon DMLC and VMAT (Craft et al. Plan averaging for multicriteria navigation of sliding window IMRT and VMAT, Medical Physics 41(2), 021709, 2014).

For passive protons, MCO with a mimicking step can be used for any delivery technique. Directly deliverable MCO can be used in the following settings:

- DMLC with predetermined or optimized SOBP. All plans must have the same shape of the SOBPs, but the shapes can be different for different beams, and different plans can scale the weight of each beam individually.

- SMLC with the same segments in all plans, but where each plan (and even each segment) can have individual SOBPs A simple variant is to have plans with blocks and only one segment per beam, but different SOBPs

Fig. 3 is a flow chart defining the steps of the inventive method. In step S31, an optimization problem is defined, in the manner described above. The optimization problem is set up to allow variation in settings of the range modulating device, and/or settings of the aperture element during the delivery of the first beam, so that said plan will include modulation of the fluence of the beam during the delivery of the beam. In step S32, the optimization problem is used to optimize a treatment plan.

Fig. 4 is a schematic representation of a computer system in which the inventive method may be performed. A computer 51 comprises a processor 53, a data memory 54 and a program memory 55. Preferably, a user input means 58 is also present, in the form of a keyboard, a mouse, a joystick, voice recognition means or any other available user input means.

The data memory is arranged to hold data for use in the method, such as variable settings.

As will be understood, the data memory 54 is only shown schematically. There may be several data memory units, each holding one or more different types of data, for example, one data memory for the value set, one for the objective function, etc.

The program memory 55 holds a computer program arranged to control the processor to perform the optimization according to the embodiments of the invention.

**Claims**

1. A method of optimizing a radiotherapy plan for passive ion therapy designed to expose a patient to ion radiation delivered as at least a first beam delivered from a first beam angle, where each beam is shaped by means of passive devices including: a range modulating device (9) for creating a spread out Bragg peak, SOBP and an aperture element (11) for shaping the beam laterally, using an optimization problem set up to allow variation in settings of the range modulating device, and settings of the aperture element both during the delivery of the first beam from the first beam angle, so that said plan will include modulation of the fluence of the beam during the delivery of the beam.

2. A method according to claim 1, wherein the optimization problem is set up in such a way that the resulting radiotherapy plan is designed to deliver ion radiation in at least a first beam and a second beam, from a first and a second beam angle, respectively, the method including the step of modulating the fluence of each beam during the delivery of the beam.

3. A method according to claim 1 or 2, wherein the optimization problem is set up to allow variation of the settings of the aperture element (11) during the delivery of the at least first beam, so that said plan will include lateral modulation of the beam during the delivery of the beam.

4. A method according to claim 3, wherein the optimization problem is set up to optimize at least the first beam for delivery in at least a first and a second segment and to return a plan where the settings for the aperture element (11) are different for the first and second segment.

5. A method according to claim 3, wherein the optimization problem is set up to optimize the at least first beam wherein control points are used to define at least a first and a second portion of the at least first beam and the settings for the aperture element (11) are different for the first and the second portion.

6. A method according to any one of the preceding claims, wherein the optimization problem is set up to allow variation of the range modulating device (9) during the delivery of the at least first beam, so that said plan will include modulation in depth of the beam during the delivery of the beam.

7. A method according to claim 6, wherein the optimization problem is set up to optimize at least the first beam for delivery in at least a first and a second segment and to return a plan where the settings for the range modulating device (11) are different for the first and second segment.

8. A method according to claim 6, wherein the optimization problem is set up to optimize the at least first beam wherein control points are used to define at least a first and a second portion of the at least first beam and the settings for the range modulating device (9) are different for the first and the second portion.

9. A method according to any one of the preceding claims, wherein the passive devices include a range compensator (15) and the optimization is set up to allow variation in settings of the range compensator (15).

10. A computer program product comprising computer readable code means, which, when run in a computer, will cause the computer to perform the method according to any one of the preceding claims.

11. A computer system (51) for performing dose calculations for radiotherapy, the system comprising processing means (53), said computer system having a program memory (55) having stored therein a computer program product according to claim 10 in such a way that the computer program product, when executed, will control the processing means (53).

12. A radiotherapy delivery system for delivering a passive ion plan wherein ions are delivered to a patient in at least a first beam delivered from a first beam angle, the system comprising a range modulating device (9) for creating a spread-out Bragg peak, SOBP, and an aperture element (11) for shaping the beam laterally, **characterised by** the system further comprising control means (16) for varying the settings of the aperture element (11) and the settings of the range modulating device (9) both during delivery of the at least first beam from the first beam angle, so as to modulate the fluence of the beam during delivery of the beam.

13. A system according to claim 12, wherein the aperture element (11) comprises an element that is capable of varying the lateral shape of the beam, and the control means (16) is arranged to control the aperture element (11) so as to modulate the at least one beam laterally by using multiple settings of the aperture element (11) for at least one beam angle.

14. A system according to claim 12 or 13, wherein the control means (16) is arranged to control the range modulating device (9) to vary the SOBP width in such a way that at least two different SOBT widths are applied to the same beam.

15. A system according to any one of the claims 12 - 14, further comprising a range compensator (15), wherein the control means (16) is arranged to control the range compensator (15) to vary the beam range in such a way that at least two different beam ranges are applied to the same beam.

**Patentansprüche**

1. Verfahren zum Optimieren eines Strahlentherapieplans für eine passive Ionentherapie, die konzipiert ist, um einen Patienten einer Ionenstrahlung auszusetzen, die als mindestens ein erster Strahl abgegeben wird, der aus einem ersten Strahlwinkel abgegeben wird, wobei jeder Strahl mittels passiver Vorrichtungen geformt wird, einschließlich: einer Bereichsmodulationsvorrichtung (9) zum Erzeugen eines ausgebreiteten Bragg-Peaks, SOBP, und eines Blendenelements (11) zum seitlichen Formen des Strahls, unter Verwendung eines Optimierungsproblems, das eingerichtet ist, um eine Variation in Einstellungen der Bereichsmodulationsvorrichtung und Einstellungen des Blendenelements, beide während der Abgabe des ersten Strahls aus dem ersten Strahlwinkel, zu ermöglichen, so dass der Plan eine Modulation der Fluenz des Strahls während der Abgabe des Strahls einschließt.

2. Verfahren nach Anspruch 1, wobei das Optimierungsproblem derart eingerichtet ist, dass der resultierende Strahlentherapieplan konzipiert ist, um Ionenstrahlung in mindestens einem ersten Strahl und einem zweiten Strahl, aus einem ersten beziehungsweise einem zweiten Strahlwinkel abzugeben, wobei das Verfahren den Schritt des Modulierens der Fluenz jedes Strahls während der Abgabe des Strahls einschließt.

3. Verfahren nach Anspruch 1 oder 2, wobei das Optimierungsproblem eingerichtet ist, um eine Variation der Einstellungen des Blendenelements (11) während der Abgabe des mindestens ersten Strahls zu ermöglichen, sodass der Plan eine seitliche Modulation des Strahls während der Abgabe des Strahls einschließt.

4. Verfahren nach Anspruch 3, wobei das Optimierungsproblem eingerichtet ist, um mindestens den ersten Strahl für die Abgabe in mindestens einem ersten und einem zweiten Segment zu optimieren und einen Plan zurückzugeben, in dem die Einstellungen für das Blendenelement (11) für das erste und das zweite Segment unterschiedlich sind.

5. Verfahren nach Anspruch 3, wobei das Optimierungsproblem eingerichtet ist, um den mindestens ersten Strahl zu optimieren, wobei Kontrollpunkte verwendet werden, um mindestens einen ersten und einen zweiten Abschnitt des mindestens ersten Strahls zu definieren, und wobei die Einstellungen für das Blendenelement (11) für den ersten und den zweiten Abschnitt unterschiedlich sind.

6. Verfahren nach einem der vorstehenden Ansprüche, wobei das Optimierungsproblem eingerichtet ist, um eine Variation der Bereichsmodulationsvorrichtung (9) während der Abgabe des mindestens ersten Strahls zu ermöglichen, sodass der Plan eine Modulation in der Tiefe des Strahls während der Abgabe des Strahls einschließt.

7. Verfahren nach Anspruch 6, wobei das Optimierungsproblem eingerichtet ist, um mindestens den ersten Strahl für die Abgabe in mindestens einem ersten und einem zweiten Segment zu optimieren und einen Plan zurückzugeben, in dem die Einstellungen für die Bereichsmodulationsvorrichtung (11) für das erste und das zweite Segment unterschiedlich sind.

8. Verfahren nach Anspruch 6, wobei das Optimierungsproblem eingerichtet ist, um den mindestens ersten Strahl zu optimieren, wobei Kontrollpunkte verwendet werden, um mindestens einen ersten und einen zweiten Abschnitt des mindestens ersten Strahls zu definieren, und wobei die Einstellungen für die Bereichsmodulationsvorrichtung (9) für den ersten und den zweiten Abschnitt unterschiedlich sind.

9. Verfahren nach einem der vorstehenden Ansprüche, wobei die passiven Vorrichtungen einen Bereichskompensator (15) einschließen und die Optimierung eingerichtet ist, um eine Variation in Einstellungen des Bereichskompensators (15) zu ermöglichen.

10. Computerprogrammprodukt, umfassend computerlesbare Codemittel, die, wenn sie auf einem Computer ablaufen, den Computer veranlassen, das Verfahren nach einem der vorstehenden Ansprüche durchzuführen.

11. Computersystem (51) zum Durchführen von Dosisberechnungen für die Strahlentherapie, das System umfassend Verarbeitungsmittel (53), wobei das Computersystem einen Programmspeicher (55) aufweist, in dem ein Computerprogrammprodukt nach Anspruch 10 derart gespeichert ist, dass das Computerprogrammprodukt, wenn es ausgeführt wird, die Verarbeitungsmittel (53) steuert.

12. Strahlentherapieabgabesystem zum Abgeben eines passiven Ionenplans, wobei Ionen an einen Patienten in mindestens einem ersten Strahl abgegeben werden, der aus einem ersten Strahlwinkel abgegeben wird, das System umfassend eine Bereichsmodulationsvorrichtung (9) zum Erzeugen eines ausgebreiteten Bragg-Peaks, SOBP, und ein Blendenelement (11) zum seitlichen Formen des Strahls, **gekennzeichnet durch**
das System ferner umfassend ein Steuermittel (16) zum Variieren der Einstellungen des Blendenelements (11) und der Einstellungen der Bereichsmodulationsvorrichtung (9), beide während der Abgabe des mindestens ersten Strahls aus dem ersten Strahlwinkel, um die Fluenz des Strahls während der Abgabe des Strahls zu modulieren.

13. System nach Anspruch 12, wobei das Blendenelement (11) ein Element umfasst, das in der Lage ist, die seitliche Form des Strahls zu variieren, und wobei das Steuermittel (16) angeordnet ist, um das Blendenelement (11) zu steuern, um den mindestens einen Strahl seitlich zu modulieren, durch Verwenden mehrere Einstellungen des Blendenelements (11) für mindestens einen Strahlwinkel.

14. System nach Anspruch 12 oder 13, wobei das Steuermittel (16) angeordnet ist, um die Bereichsmodulationsvorrichtung (9) zu steuern, um die SOBP-Breite derart zu variieren, dass mindestens zwei unterschiedliche SOBT-Breiten auf denselben Strahl angewendet werden.

15. System nach einem der Ansprüche 12 bis 14, ferner umfassend einen Bereichskompensator (15), wobei das Steuermittel (16) angeordnet ist, um den Bereichskompensator (15) zu steuern, um den Strahlbereich derart zu variieren, dass mindestens zwei unterschiedliche Strahlbereiche auf denselben Strahl angewendet werden.

**Revendications**

1. Procédé d'optimisation d'un plan de radiothérapie pour thérapie ionique passive conçu pour exposer un patient à un rayonnement ionique administré en tant qu'au moins un premier faisceau administré à partir d'un premier angle de faisceau, où chaque faisceau est formé au moyen de dispositifs passifs comportant : un dispositif de modulation de plage (9) permettant de créer un pic de Bragg étalé (SOBP) et un élément d'ouverture (11) permettant de former le faisceau de manière latérale, à l'aide d'un problème d'optimisation établi pour permettre une variation de réglages du dispositif de modulation de plage, et des réglages de l'élément d'ouverture à la fois pendant l'administration du premier faisceau à partir du premier angle de faisceau, de sorte que ledit plan comporte une modulation de la fluence du faisceau pendant l'administration du faisceau.

2. Procédé selon la revendication 1, dans lequel le problème d'optimisation est établi de manière à ce que le plan de radiothérapie résultant soit conçu pour administrer un rayonnement ionique dans au moins un premier faisceau et un second faisceau, à partir d'un premier et d'un second angle de faisceau, respectivement, le procédé comportant l'étape de modulation de la fluence de chaque faisceau pendant l'administration du faisceau.

3. Procédé selon la revendication 1 ou 2, dans lequel le problème d'optimisation est établi pour permettre une variation des réglages de l'élément d'ouverture (11) pendant l'administration de l'au moins un premier faisceau, de sorte que ledit plan comporte une modulation latérale du faisceau pendant l'administration du faisceau.

4. Procédé selon la revendication 3, dans lequel le problème d'optimisation est établi pour optimiser au moins le premier faisceau d'administration dans au moins un premier et un second segment et pour renvoyer un plan où les réglages de l'élément d'ouverture (11) sont différents pour les premier et second segments.

5. Procédé selon la revendication 3, dans lequel le problème d'optimisation est établi pour optimiser l'au moins un premier faisceau, dans lequel des points de commande sont utilisés pour définir au moins une première et une seconde partie de l'au moins un premier faisceau et les réglages de l'élément d'ouverture (11) sont différents pour la première et la seconde partie.

6. Procédé selon l'une quelconque des revendications précédentes, dans lequel le problème d'optimisation est établi pour permettre une variation du dispositif de modulation de plage (9) pendant l'administration de l'au moins un premier faisceau, de sorte que ledit plan comporte une modulation de profondeur du faisceau pendant l'administration du faisceau.

7. Procédé selon la revendication 6, dans lequel le problème d'optimisation est établi pour optimiser au moins le premier faisceau pour l'administration dans au moins un premier et un second segment et pour renvoyer un plan où les réglages du dispositif de modulation de plage (11) sont différents pour les premier et second segments.

8. Procédé selon la revendication 6, dans lequel le problème d'optimisation est établi pour optimiser l'au moins un premier faisceau, dans lequel des points de commande sont utilisés pour définir au moins une première et une seconde partie de l'au moins un premier faisceau et les réglages du dispositif de modulation de plage (9) sont différents pour la première et la seconde partie.

9. Procédé selon l'une quelconque des revendications précédentes, dans lequel les dispositifs passifs comportent un compensateur de plage (15) et l'optimisation est établie pour permettre une variation des réglages du compensateur de plage (15).

10. Produit programme informatique comprenant un moyen de code lisible par ordinateur qui, lorsqu'il est exécuté dans un ordinateur, amènera l'ordinateur à réaliser le procédé selon l'une quelconque des revendications précédentes.

11. Système informatique (51) permettant d'effectuer des calculs de dose pour une radiothérapie, le système comprenant un moyen de traitement (53), ledit système informatique ayant une mémoire de programme (55) sur laquelle est stocké un produit de programme informatique selon la revendication 10 de telle sorte que le produit de programme informatique, lorsqu'il est exécuté, commande le moyen de traitement (53).

12. Système d'administration de radiothérapie permettant d'administrer un plan ionique passif, dans lequel des ions sont administrés à un patient dans au moins un premier faisceau administré à partir d'un premier angle de faisceau, le système comprenant un dispositif de modulation de plage (9) permettant de créer un pic de Bragg étalé (SOBP) et un élément d'ouverture (11) permettant de former le faisceau de manière latérale, **caractérisé par**
le système comprenant en outre un moyen de commande (16) permettant de faire varier les réglages de l'élément d'ouverture (11) et les réglages du dispositif de modulation de plage (9) à la fois pendant l'administration de l'au moins un premier faisceau à partir du premier angle de faisceau, de façon à moduler la fluence du faisceau pendant l'administration du faisceau.

13. Système selon la revendication 12, dans lequel l'élément d'ouverture (11) comprend un élément qui est capable de faire varier la forme latérale du faisceau, et le moyen de commande (16) est agencé pour amener l'élément d'ouverture (11) à moduler l'au moins un faisceau de manière latérale en utilisant plusieurs réglages de l'élément d'ouverture (11) pour au moins un angle de faisceau.

14. Système selon la revendication 12 ou 13, dans lequel le moyen de commande (16) est agencé pour amener le dispositif de modulation de plage (9) à faire varier la largeur de SOBP de manière à ce qu'au moins deux largeurs de SOBT différentes soient appliquées au même faisceau.

15. Système selon l'une quelconque des revendications 12 à 14, comprenant en outre un compensateur de plage (15), dans lequel le moyen de commande (16) est agencé pour amener le compensateur de plage (15) à faire varier la plage de faisceaux de telle sorte qu'au moins deux plages de faisceaux différentes soient appliquées au même faisceau.

# FIGURE 1

# FIGURE 2

# FIGURE 3

# FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 15173971 A
- US 2017281974 A

- EP 18183065

### Non-patent literature cited in the description

- **THIEKE et al.** A new concept for interactive radiotherapy planning with multicriteria optimization: First clinical evaluation. *Radiotherapy and Oncology,* 2007, vol. 85 (2), 292-298

- **CRAFT et al.** An approach for practical multiobjective IMRT treatment planning. *International Journal of Radiation Oncology\*Biology\*Physics,* 2007, vol. 69 (5), 1600-1607
- **CRAFT et al.** Plan averaging for multicriteria navigation of sliding window IMRT and VMAT. *Medical Physics,* 2014, vol. 41 (2), 021709